# EUROPEAN PATENT SPECIFICATION

(11) **EP 0 389 437 B1**
(45) Date of publication and mention of the grant of the patent: **24.05.1995**
(21) Application number: 90810220.5
(22) Date of filing: 20.03.1990
(51) Int. Cl.: C07D 487/04, C09K 11/06

(54) **Luciferin derivatives**
Luciferinderivate
Dérivés de la luciférine

(30) Priority: 22.03.1989 JP 71037/89
(43) Date of publication of application: 26.09.1990
(73) Proprietor: NIPPON ZOKI PHARMACEUTICAL CO. LTD., Osaka (JP)
(72) Inventor: Okamoto, Kaoru, c/oInstitute of Bio-Active Science, Yashiro-cho, Katoh-gun, Hyogo (JP); Goto, Toshio, Nagoya (JP)
(74) Representative: Kerr, Andrew

(56) References cited:
- EP-A- 0 137 515
- BIOCHEMISTRY, vol. 18, 1979 R.C. HART et al. "Renilla reniformisBioluminescence: Luciferase-Catalyzed Pro- duction of Nonradiating Ex- citedStates from Luciferin Analogues and Elucidation of the Excited State SpeciesInvolved in Energy Transfer to Renilla Green Fluorescent Protein" pages 2204-2210
- FEBS LETTERS, vol. 239, 1988 H. KIMURA et al. "Highly sensitive and reliable chemiluminescence method for the assay of superoxide dismutase in humanerythrocytes" pages 347-350
- B. M. Krasovitskii, B. M. Bolotin, Organic Luminescent materials. Verlag Chemie (1988).

## Description

The present invention relates to derivatives of luciferin analogues and salts thereof which are useful as chemiluminescent reagents and to novel intermediates useful in their preparation.

In recent years, chemiluminescence microanalyses of the substances present in a living body have often been used, and many chemiluminescent reagents such as luminol derivatives and oxalic esters have been developed and used. After chemiluminescent properties were discovered in luciferins, which became known as bioluminescent substances, various kinds of chemiluminescent compounds specific for detecting active oxygen and the like were synthesized. However, when detecting active oxygen in living organisms, interference by contaminating substances having absorption or fluorescence in the visible region often impedes analysis. It is therefore an object of the invention to provide chemiluminescent reagents emitting at longer than conventional luminescent wavelenghts.

It is a further object of the invention to provide intermediates and salts thereof which are useful in the preparation of such chemiluminescent reagents.

In FEBS Letters, vol.239, No. 2 (November 1988) pages 347-350 there is described a luminescence method for the determination of oxygen generation in activated leukocyte systems using 2-methyl-6-phenyl-3,7-dihydroimidazo[1,2-a]pyrazin-3-one.

According to one aspect of the invention there are provided compounds represented by the following formula (I): 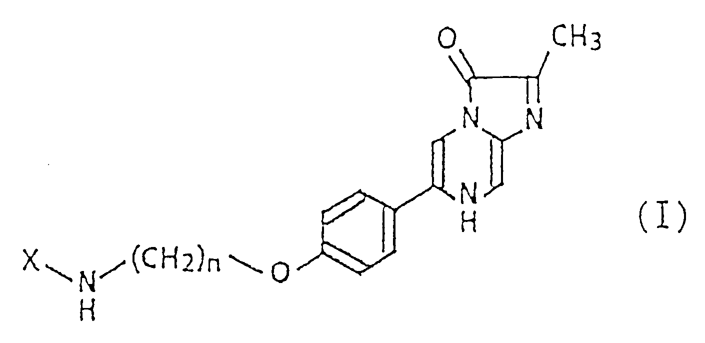
wherein X is hydrogen, an amino protecting group of the type employed in peptide synthesis, R-, R-CO-, R-SO₂-, R-NHCO-, or R-NHCS-, R is a fluorescent group selected from 5- or 6- carboxyfluorescein succinimidyl ester,
5- or 6- carboxyfluorescein-amidocaproic acid-N-hydroxysuccinimidyl ester,
fluorescein-5-isocyanate,
fluorescein-5-isothiocyanate,
5-(4,6-dichlorotriazin-2-yl)amino-fluorescein dihydrochloride,
4-dimethylamino-azobenzene-4'-sulfonyl chloride,
4-dimethylamino-azobenzene-4'-isothiocyanate,
4-chloro-7-nitrobenz-2-oxa-1,3-diazole, and
4-fluoro-7-nitrobenz-2-oxa-1,3-diazole
and n is an integer from 1 to 4, or salts thereof.

The above novel compounds include both chemiluminescence reagents and intermediates for their preparation. Thus the invention includes a chemiluminescence reagent, being a compound as defined above wherein wherein X in formula (I) is R-, R-CO-, R-SO₂-, R-NHCO-, or R-NHCS-, and R is a said fluorescent group, or a salt thereof.

The fluorescent group R in formula (I) above preferably exhibits an excited wavelength spectrum substantially overlapping that of 3,7-dihydro-6-(4-methoxyphenyl)-2-methylimidazo[1,2-a]pyrazin-3-one. Thus it should have an emission wavelength from 400 to 530 nm or more preferably from 470 to 510 nm. R may, for example be derived from fluorescein, 4-dimethylaminophenylazobenzene or 4-nitrobenz-2-oxa-1,3-diazole.

The invention is inclusive of the use of a chemiluminescence reagent as defined above in the determination of active oxygen in living organisms.

The invention also includes an intermediate for the production of a chemiluminescence reagent as defined above which is a compound according to the invention wherein X is hydrogen or an amino protecting group of the type employed in peptide synthesis, e.g.:
benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenyl-azobenzyloxycarbonyl, t-butoxycarbonyl, t-amyloxycarbonyl, p-biphenylisopropyloxycarbonyl, diisopropylmethyloxycarbonyl and formyl. The compounds of the present invention include salts of the compounds having formula (I) above, for example, salts with alkali metal such as sodium, potassium or lithium, salts with alkaline-earth metal such as magnesium, calcium or barium, salts with other metals such as aluminium, or sats as acid addition with an acid such as hydrochloric acid, sulfuric acid, nitric acid, phosphoric acid, formic acid, acetic acid, citric acid, lactic acid, hydrobromic acidor trifluoroacetic acid, or salts with bases such as ammonia or organic amines.

These salts can be produced from the free derivatives of formula (I) in the usual way or can be interchanged with each other.

The various compounds of the present invention may be prepared as follows.

Compounds represented by the formula (II): 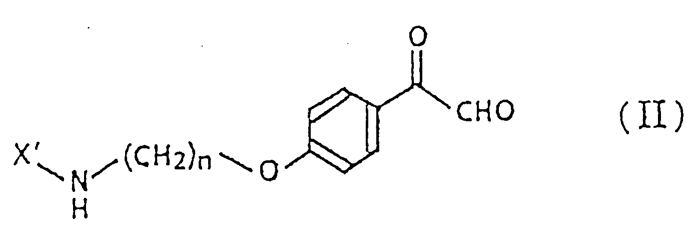
wherein X' is an amino-protecting group, and n represents an integer of 1 to 4 are condensed with 2-aminoacetamidine in an aqueous solution of potassium hydroxide to convert the said glyoxal compounds into pyrazine derivatives. The resulting products are reacted in methanol with an aqueous solution of methylglyoxal to give 3,7-dihydro[1,2-a]pyrazin-3-one derivatives corresponding to the present compounds of the formula (I) wherein X is an amino-protecting group.

Amino-protecting groups can be removed from amino-protected 3,7-dihydro[1,2-a]pyrazin-3-one derivatives to give the compounds of the present invention of the formula (I) wherein X is hydrogen. For example, in the case that benzyloxycarbonyl is used as the amino-protecting group, the protecting group can be removed by a conventional method such as a catalytic reduction, or a treatment with hydrogen bromide-acetic acid.

Various kinds of substituents can be introduced into the amino position of the said compounds from which the protecting group is removed. Therefore, the compounds are very useful as starting materials to introduce a fluorescent group into a luciferin analogue structure.

Subsequently, a fluorescent reactant having a functional group which can combine with amines can be condensed with the compounds of the present invention of the formula (I) wherein X is hydrogen to give the chemiluminescent substances of the present invention of the formula (I) wherein X is R-, R-CO, R,SO₂, R-NHCO- or R-NHCS- (R represents a fluorescent reactant). As the condensation method with amines, a replacement reaction of an elimination group such as halogen, a condensation reaction between acid halide and active ester conventionally employed in peptide synthesis, a reaction with isocyanate or isothicyanate and the like can be employed. The condensation reactions can be carried out according to the reaction conditions conventionally employed in the field of peptide synthesis and fluorescence labeling of amines. The fluorescent reactant having functional groups which can combine with amines, for example, 5- or 6-carboxy-fluorescein succinimidyl ester, 5- or 6-carboxyfluorescein-amidocaproic acid N-hydroxy-succinimide ester, fluorescein-5-isocyanate, fluorescein-5-isothiocyanate, 5-(4,6-dichlorotriazin-2-yl)amino-fluorescein dihydrochloride, 4-dimethylaminoazobenzene-4'-sulfonyl chloride, 4-dimethylaminoazobenzene-4'-isothio-cyanate, 4-chloro-7-nitrobenz-2-oxa-1,3-diazole, 4-fluoro-7-nitrobenz-2-oxa-1,3-diazole and the like can be employed.

The resulting compounds of the present invention can be purified by known methods such as distillation chromatography and recrystallization. Identification is established through, inter alia, melting point, elemental analysis, IR, NMR, UV, mass spectrum, etc.

The invention will now be illustrated with reference to the following Examples.

### Example 1.

(1) To ice-cold solution of 2-aminoethanol (2.1 mol) in 50% ethanol (800 ml), 100 ml of benzyl chloroformate was slowly added. After stirring for 2 hrs at the same temperature, the mixture was concentrated to the half volume. The residue was acidified (pH 2) with 1N sulfuric acid and extracted three times with chloroform. The extracts were washed with water, dried over anhydrous sodium sulfate, and evaporated to give an oil, which was crystallized from ethanol to give 115 g of 2-(N-benzyloxycarbonylamino)ethanol as white needles.
   m.p.: 55 - 57 °C
   IR (KBr): 3330, 1692, 1540, 1275, 1210, 1143, 1032, 993, 742, 695 cm⁻¹
   MS (EI, 20eV): m/z 195 (M⁺)
   NMR (CDCl₃): δ=2.87(1H,br.s), 3.25-3.35(2H,m), 3.66(2H,t,J=4.6Hz), 5.08(2H,s), 5.42(1H,br), 7.25-7.40(5H,m)
(2) To a solution of the resulting product (28 g) in pyridine (200 ml), 25 g of p-toluenesulfonyl chloride was added. The mixture was stirred at 0°C for 1 hr, poured into ice-water, acidified with 6N HCl, and extracted with ethyl acetate. The extracts were washed with water and brine, dried over anhydrous sodium sulfate, and evaporated to give 43 g of 2-(N-benzyloxycarbonylamino)ethyl p-toluenesulfonate as an oil.
   IR (Neat): 3350, 1730, 1498, 1455, 1215, 1160, 1121, 1080, 1035, 1021, 816, 738, 685 cm⁻¹
   NMR (CDCl₃): δ=2.43(3H,s), 3.44(2H,dt,J=5.0, 5.3Hz), 4.08(2H,t,J=5,0Hz), 5.05(2H,s), 5.17(1H,br.t), 7.25-7.45(5H,m), 7.32(2H,d,J=9.0Hz), 7.77(2H,d,J=9.0Hz)
(3) A mixture of 35 g of 2-(N-benzyloxycarbonylamino)ethyl p-toluenesulfonate, 13 g of 4′-hydroxyacetophenone and 55 g of anhydrous potassium carbonate in 450 ml of dry acetone was refluxed with stirring for 24 hrs under argon atmosphere. After cooling, the insoluble material was removed by filtration and the filtrate was evaporated. The residue was diluted with ethyl acetate, washed with water, 1% NaOH and brine, dried over anhydrous sodium sulfate, and evaporated under reduced pressure to give a solid, which was recrystallized from hexane-benzene to give 26.5 g of 4′-(2-(N-benzyloxycarbonylamino)ethoxy)acetophenone as white needles.
   m.p.: 80 - 81 °C
   IR (KBr): 3310, 1690, 1666, 1600, 1560, 1356, 1273, 1257, 1165, 1112, 1055, 837, 826, 750, 698 cm⁻¹
   MS (EI, 20eV): m/z 313 (M⁺)
   NMR (CDCl₃): δ=2.55(3H,s), 3.63(2H,dt,J=5.0, 5.3Hz), 4.10(2H,t,J=5.0Hz), 5.11(2H,s), 5.27(1H,br.t), 6.90(2H,d,J=8.7Hz), 7.27-7.42(5H,m), 7.92(2H,d,J=8.7Hz)
(4) A mixture of 21 g of the resulting product, 7.44 g of selenium dioxide, 1.35 ml of water and 34 ml of dioxane was refluxed for 20 hrs. After removal of selenium, the solvent was evaporated to give a viscous oil, which was treated with 300 ml of water at 100°C for 3 hrs with stirring. The solution was concentrated to give 23 g of 4-(2-(N-benzyloxycarbonylamino)ethoxy)phenylglyoxal hydrate as an oil, which was used in the next reaction without further purification.
(5) To a solution of 4-(2-(N-benzyloxycarbonylamino)ethoxy)phenylglyoxal hydrate (16 g) in methanol (60 ml) and 1,4-dioxane (60 ml), a solution of 2-aminoacetamidine dihydrobromide (11 g) in water was added at -15°C. A 20% KOH solution (32 ml) was added thereto and the mixture was stirred at -15°C for 1 hr and at room temperature for 1 hr. The solvent was removed under reduced pressure and the residue was dried to give a crude solid, which was triturated with 2-propanol. The insoluble solid was collected by filtration, washed with cold 2-propanol, and dried to give 5.9 g of 2-amino-5-(4-(2-(N-benzyloxycarbony-lamino)ethoxy)phenyl)pyrazine as pale yellow crystals.
   m.p.: 128 - 130 °C
   IR (KBr): 3380, 3175, 1686, 1601, 1522, 1504, 1478, 1458, 1259, 1250, 1193, 1140, 1010, 820, 696 cm⁻¹
   MS (EI, 20eV): m/z 364 (M⁺)
   NMR (CDCl₃): δ=3.63(2H,dt,J=5.0, 5.2Hz), 4.08(2H,t,J=5.0Hz), 4.61(2H,br.s), 5.13(2H,s), 5.35(1H,br.t), 6.94(2H,d,J=8.7Hz), 7.26-7.42(5H,m), 7.78(2H,d,J=8.7Hz), 8.02(1H,d,J=1.4Hz), 8.36(1H,d,J=1.4Hz)
(6) The resulting product (1.0 g) and methylglyoxal (5.55 mmol) were dissolved in 23 ml of methanol, and 0.55 ml of conc hydrochloric acid was added thereto at room temperature under argon atmosphere. The mixture was stirred at 70°C for 4 hrs and condensed to a crude powder, which was triturated with water. The precipitated solid was filtered, washed with water and ether, and dried to give 1.2 g of 6-(4-(2-(N-benzyloxycarbonylamino)ethoxy)phenyl)-3,7-dihydro-2-methylimidazo[1,2-a]pyrazin-3-one hydrochloride as yellow crystals.
   m.p.: 152 - 155 °C
   IR (KBr): 3350, 3100-2000, 1700, 1652, 1598, 1500, 1241, 830 cm⁻¹
   MS (SIMS): m/z 419 (M+H)
   NMR (CDCl₃): δ=2.56(3H,s), 3.54(2H,t,J=5.6Hz), 4.11(2H,t,J=5.6Hz), 5.09(2H,s), 7.08(2H,d,J=8.6Hz), 7.22-7.40(5H,m), 7.89(2H,d,J=8.6Hz), 8.53(1H,d,J=1.1Hz), 9.04(1H,d,J=1.1Hz)
(7) A mixture of the resulting product (500 mg) and anhydrous hydrogen bromide in acetic acid (30% solution, 1.2 ml) was stirred at room temperature for 10 minutes. Ether was added to the mixture and the precipitated solid was filtered, washed with ether, followed by chloroform, and dried to give 490 mg of 6-(4-(2-aminoethoxy)phenyl)3,7-dihydro-2-methylimidazo[1,2-a]pyrazine-3-one dihydrobromide as a hygroscopic powder.
   MS (SIMS): m/z 285 (M+H)
   NMR (CD₃OD): δ=2.58(3H,s), 3.44(2H,t,J=4.9Hz), 4.36(2H,t,J=4.9Hz), 7.21(2H,d,J=8.7Hz), 8.01(2H,d,J=8.7Hz), 8.65(1H,br.s), 9.16(1H,br.s)
(8) The resulting product (525 mg) was dissolved in 10 ml of water, and a 5% solution of sodium hydrogencarbonate was added thereto until the pH 9. 460 mg of fluorescein isothiocyanate in acetone was added, and the mixture was stirred for 2 hrs. The reaction was stopped by acidifying with acetic acid to pH 4.5, and the precipitated solid was collected by filtration, washed with water, acetone, methanol followed by ether to give 450 mg of 3,7-dihydro-6-(4-(N′-(5-fluoresceinyl)thioureido)ethoxy)-phenyl)-2-methylimidazo[1,2-a]pyradine-3-one (Compound 1) as yellow crystals. 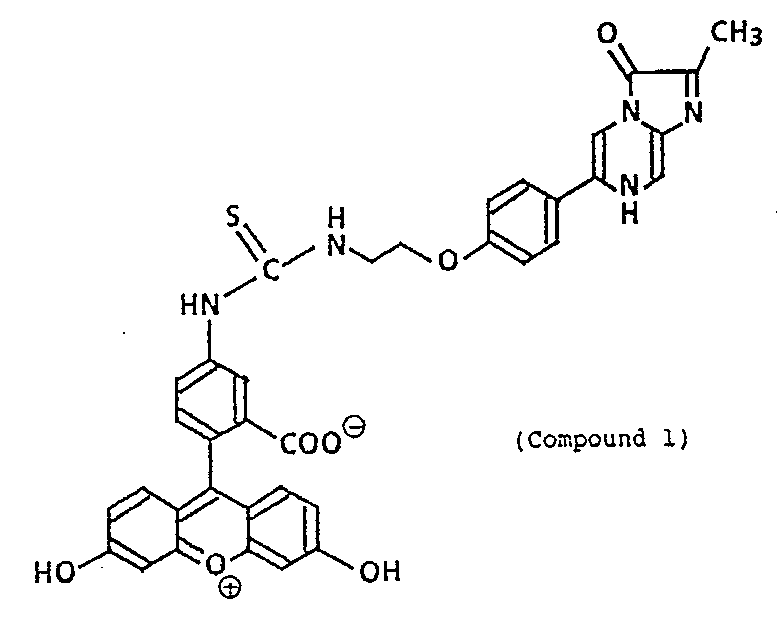 m.p.: 226 - 228 °C (decomposition)
   IR (KBr): 3700-2000, 1602, 1580, 1505, 1461, 1245, 1175, 1106, 835 cm⁻¹
   MS (SIMS): m/z 674 (M+H)
   UV: λₘₐₓ=284, 355, 452 nm (in MeOH)
   λₘₐₓ=253, 265, 440 nm (in MeOH-HCl)
   λₘₐₓ=240, 265, 495 nm (in MeOH-NaOH)
   NMR (CDCl₃/CD₃OD=l/l): δ=2.50(3H,s), 4.14(2H,t,J=5.6Hz), 4.32(2H,t,J=5.6Hz), 6.55(2H,dd,J=2.4, 8.6Hz), 6.70(2H,d,J=2.5Hz), 6.72(2H,d,J=8.6Hz), 7.10(2H,d,J=10.9Hz), 7.15(1H,d,J=8.3Hz), 7.58(2H,d,J=10.9Hz), 7.59(1H,br.s), 7.73(1H,br.s), 7.87(1H,dd,J=2.1, 8.3Hz), 8.11(1H,d,J=2.1Hz)

### Example 2.

Compound 1 (1.8 g) was suspended in methanol, and 225 mg of sodium hydrogencarbonate in water (90 ml) was added thereto at room temperature. After stirring at room temperature for 30 minutes, the solution was warmed until becoming transparent and condensed under reduced pressure. To the residue, 20 ml of methanol and 100 ml of ether were added. The precipitated solid was collected by filtration, washed with ether, and dried to give 1.71 g of sodium salt of Compound 1
m.p.: 228 - 232 °C (decomposition)
IR (KBr): 3700-2000, 1630, 1582, 1510, 1470, 1382, 1320, 1250, 1210, 1108, 850 cm⁻¹
MS (SIMS): m/z 674 (M+H), 697(M+Na+1), 719(M+2Na)
UV: λₘₐₓ=455, 485 nm (in water)
λₘₐₓ=492 nm (in water-HCl)
λₘₐₓ=443 nm (in water-NaOH)
NMR (CDCl₃/CD₃OD=l/l): δ=2.50(3H,s), 4.13(2H,t,J=5.2Hz), 4.13(2H,t,J=5.2Hz), 4.33(2H,t,J=5.2Hz), 6.78(2H,dd,J=2.2, 8.9Hz), 6.93(2H,d,J=2.2Hz), 7.01(2H,d,J=8.9Hz), 7,12(2H,d,J=8,8Hz), 7,20(1H,d,J=8,3Hz), 7,63(2H,d,J=8,8Hz), 7,70(1H,br.s), 7,29(1H,br.s), 8,00(1H,dd,J=2,0, 8,3Hz), 8,34(1H,d,J=2,0Hz)

### Measurement of Chemiluminescence Spectrum

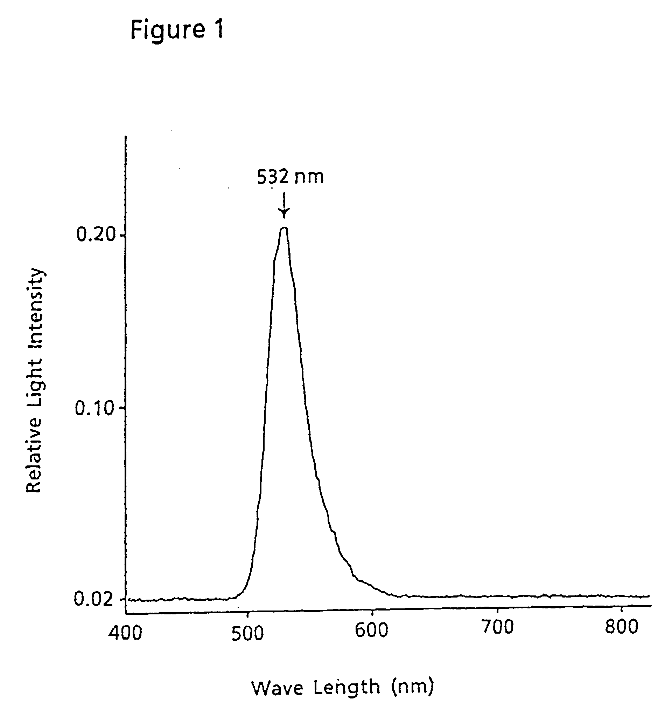

3ml of 0,01M cetyltrimethylammonium bromide in 0,1M phosphate buffer (pH 7,0) was put into a quart glass cell. About 10 g of Compound 1 of the present invention was added thereto and dissolved, and then chemiluminescence spectrum was immediately measured with a luminescence spectrometer. the result is shown in Figure 1.

Figure 1 shows emission spectrum of Compound 1 of the present invention in chemiluminescence system.

As shown by the result of the measurement of chemiluminescence spectrum in Figure 1, the peak fluorescence emitted by Compound 1 was measured at 532 nm in the chemiluminescence system. In comparison to the peak luminescence wavelength (380 nm) of 2-methyl-6-phenyl-3,7-dihydroimidazo-[1,2-a]pyrazin-3-one which is often used as a chemiluminescent reagent, Compound 1 of the present invention has 150 nm longer peak luminescence wavelenght. Therefore, it is advantageously possible to measure the emitted chemiluminescence by using the compounds of the present invention with littel interference of the living body's components having absorption or fluorescence in the visible region.

The luciferin derivatives of the present invention, which are prepared by a covalent combination of a fluorescent reactant with a luciferin compound, are novel compounds making it possible to detect active oxygen by characteristic luminescence having a longer wavelength than is conventional. Compared with conventional chemiluminescence substances, the compounds of this invention emit a luminescence of longer wavelength, so that the compounds of the present invention are very useful as chemiluminescent reagents for emission spectrochemical analyses, for example, the determination of active oxygen in living body's samples containing many interfering substances, or the analyses of various living body's component substances in combination with a in vivo system producing active oxygen and/or an enzymatic system relating to production of active oxygen.

## Claims (Claims for the following Contracting State(s): AT, BE, CH, DE, DK, FR, GB, IT, LI, LU, NL, SE)

1. Compounds represented by the following formula (I): 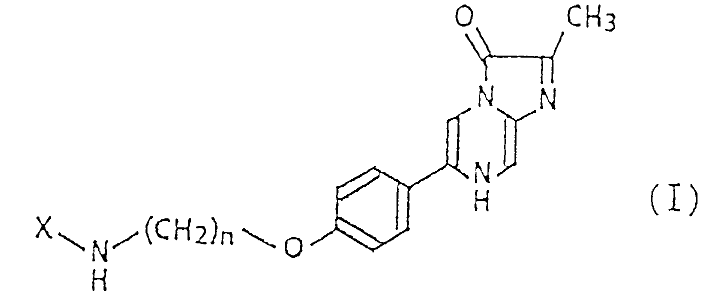 wherein X is hydrogen, an amino protecting group of the type employed in peptide synthesis, R-, R-CO-, R-SO₂-, R-NHCO-, or R-NHCS-, R is a fluorescent group selected from 5- or 6- carboxyfluorescein succinimidyl ester,
5- or 6- carboxyfluorescein-amidocaproic acid-N-hydroxysuccinimidyl ester,
fluorescein-5-isocyanate,
fluorescein-5-isothiocyanate,
5-(4,6-dichlorotriazin-2-yl)amino-fluorescein dihydrochloride,
4-dimethylamino-azobenzene-4'-sulfonyl chloride,
4-dimethylamino-azobenzene-4'-isothiocyanate,
4-chloro-7-nitrobenz-2-oxa-1,3-diazole, and
4-fluoro-7-nitrobenz-2-oxa-1,3-diazole
and n is an integer from 1 to 4, or salts thereof.

2. A chemiluminescence reagent, being a compound according to claim 1 wherein wherein X in formula (I) is R-, R-CO-, R-SO₂-, R-NHCO-, or R-NHCS-, and R is a said fluorescent group, or a salt thereof.

3. A chemiluminescence reagent according to claim 2 which is 3,7-dihydro-6-(4-(2-(N'-(5-fluoresceinyl)-thioureido)ethoxy)phenyl)-2-methylimidazo[1,2-a]pyrazin-3-one, or a salt thereof.

4. A chemiluminescence reagent according to claim 3 which is the sodium salt.

5. The use of a chemiluminescence reagent according to any of claims 2 to 4 in the determination of active oxygen in living organisms.

6. An intermediate for the production of chemiluminescence reagents according to claim 2 which is a compound according to claim 1 wherein X is hydrogen.

7. An intermediate for the production of chemiluminescence reagents according to claim 2 which is a compound according to claim 1 wherein X is an amino protecting group of the type employed in peptide synthesis.

8. An intermediate for the production of chemiluminescence reagents according to claim 2 which is a compound according to claim 1 wherein X is an amino protecting group selected from:
benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenyl-azobenzyloxycarbonyl, t-butoxycarbonyl, t-amyloxycarbonyl, p-biphenylisopropyloxycarbonyl, diisopropylmethyloxycarbonyl and formyl.

9. The use of an intermediate according to any of claims 6 to 8 for the manufacture of a chemoluminescence reagent.

10. A process for preparing an intermediate according to claim 7 or claim 8 which comprises condensing a compound of the following formula (II): 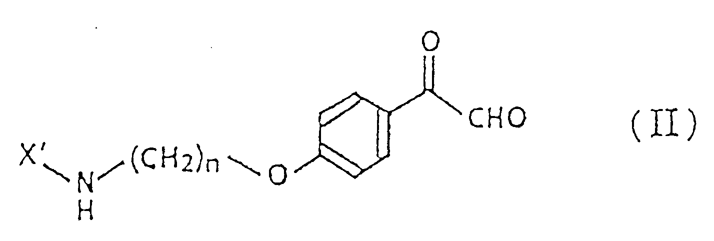 where X' is an amino-protecting group as defined in claim 7 or 8 and n is an integer from 1 to 4, with 2-aminoacetamidine to form a pyrazine derivative and reacting the resulting product with methylglyoxal to obtain a corresponding 3,7-dihydro[1,2-a]pyrazin-3-one according to formula (I) where X is a said amino-protecting group.

11. A process for preparing an intermediate according to claim 6 comprising removing an amino-protecting group from a compound according to claim 7 or 8.

12. A process for preparing an chemiluminescence reagent according to claim 2 wherein a compound according to claim 6 is reacted with a fluorescent reactant R-Y, R-CO-Y, R-SO₂-Y, R-NHCO-Y, or R-NHCS-Y, where R is a fluorescent group as defined in claim 1 and Y is an amine-reactive functional group.

## Claims (Claims for the following Contracting State(s): ES, GR)

1. A process for preparing a compound represented by the following formula (I): 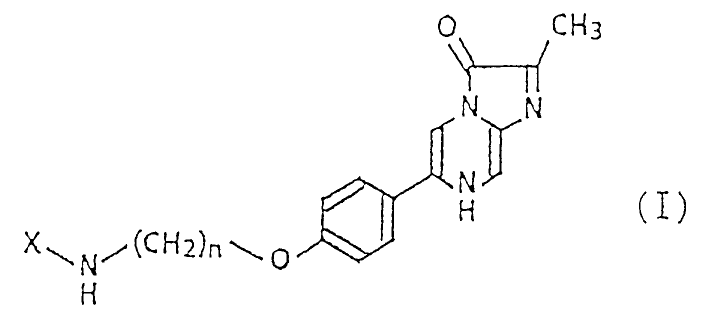 wherein X is hydrogen, an amino protecting group of the type employed in peptide synthesis, R-, R-CO-, R-SO₂-, R-NHCO-, or R-NHCS-, R is a fluorescent group selected from 5- or 6- carboxyfluorescein succinimidyl ester,
5- or 6- carboxyfluorescein-amidocaproic acid-N-hydroxysuccinimidyl ester,
fluorescein-5-isocyanate,
fluorescein-5-isothiocyanate,
5-(4,6-dichlorotriazin-2-yl)amino-fluorescein dihydrochloride,
4-dimethylamino-azobenzene-4'-sulfonyl chloride,
4-dimethylamino-azobenzene-4'-isothiocyanate,
4-chloro-7-nitrobenz-2-oxa-1,3-diazole, and
4-fluoro-7-nitrobenz-2-oxa-1,3-diazole
and n is an integer from 1 to 4, or salts thereof. which comprises:
(i) condensing a compound of the following formula (II): 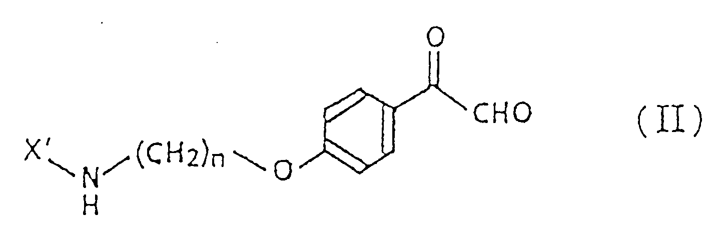 where X' is an amino-protecting group and n is an integer from 1 to 4,
with 2-aminoacetamidine to form a pyrazine derivative and reacting the resulting product with methylglyoxal to obtain a corresponding 3,7-dihydro[1,2-a]pyrazin-3-one according to formula (I) where X is a said amino-protecting group.
and
(ii) where appropriate reacting the product with a fluorescent reactant R-Y, R-CO-Y, R-SO₂-Y, R-NHCO-Y, or R-NHCS-Y, where R is a fluorescent group as defined above and Y is an amine-reactive functional group.

2. A process according to claim 1 for preparing a chemiluminescence reagent, being a compound according to formula (I) wherein X is R-, R-CO-, R-SO₂-, R-NHCO-, or R-NHCS-, and R is a said fluorescent group, or a salt thereof and wherein step (ii) is carried out.

3. A process according to claim 2 wherein said chemiluminescence reagent is 3,7-dihydro-6-(4-(2-(N'-(5-fluoresceinyl)-thioureido)ethoxy)phenyl)-2-methylimidazo[1,2-a]pyrazin-3-one, or a salt thereof.

4. A process according to claim 3 in which the sodium salt is prepared.

5. A process according to claim 1 for preparing an intermediate for the production of chemiluminescence reagents wherein X in formula (I) is hydrogen and step (ii) is not carried out.

6. A process according to claim 1 wherein X in formula (I) is an amino protecting group of the type employed in peptide synthesis and step (ii) is not carried out.

7. A process according to claim 6 wherein X is an amino protecting group selected from: benzyloxycarbonyl, p-methoxybenzyloxycarbonyl, p-chlorobenzyloxycarbonyl, p-nitrobenzyloxycarbonyl, p-phenylazobenzyloxycarbonyl, p-methoxyphenyl-azobenzyloxycarbonyl, t-butoxycarbonyl, t-amyloxycarbonyl, p-biphenylisopropyloxycarbonyl, diisopropylmethyloxycarbonyl and formyl.

8. A process according to claim 5 wherein the amino-protecting group is removed from a compound prepared according to claim 6 or claim 7.

## Patentansprüche (Patentansprüche für folgende(n) Vertragsstaat(en): AT, BE, CH, DE, DK, FR, GB, IT, LI, LU, NL, SE)

1. Verbindungen der folgenden Formel (I): 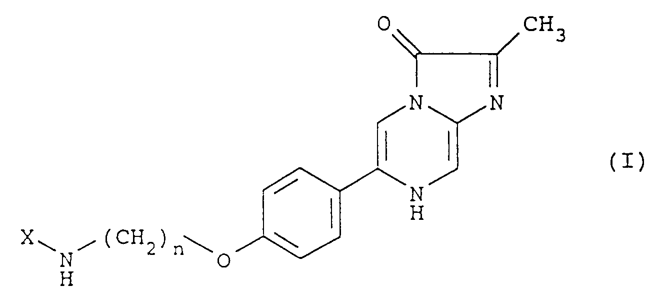 wobei X Wasserstoff, eine Aminoschutzgruppe vom in Peptidsynthesen verwendeten Typ, R-, R-CO-, R-SO₂-, R-NHCO-, oder R-NHCS-; R eine fluorenszierende Gruppe wie
5- oder 6-Carboxyfluoreszein-succinimidester,
5- oder 6-Carboxyfluoreszein-amidocapronsäure-N-hydroxysuccinimidester,
Fluoreszein-5-isocyanat,
Fluoreszein-5-isothiocyanat,
5-(4,6-Dichlorotriazin-2-yl)amino-fluoreszein dihydrochlorid,
4-Dimethylamino-azobenzyl-4'-sulfonylchlorid,
4-Dimethylamino-azobenzyl-4'-isothiocyanat,
4-Chloro-7-nitrobenz-2-oxa-1,3-diazol, und
4-Fluoro-7-nitrobenz-2-oxa-1,3-diazol,
und n ist eine Ziffer von 1 bis 4, oder Salze davon bedeutet.

2. Chemiluminiszenz-Reagens, welche eine Verbindung gemäss Anspruch 1 ist, worin X in Formel (I) R-, R-CO-, R-SO₂-, R-NHCO-, oder R-NHCS- ist, und R eine besagte fluoreszierende Gruppe, oder ein Salz davon bedeutet.

3. Chemiluminiszenz-Reagens gemäss Anspruch 2 welche 3,7-Dihydro-6-(4-(2-(N'-(5-Fluoreszeinyl)-thioureido)ethoxy)-phenyl)-2-methylimidazo [1,2-a]pyrazin-3-on, oder ein Salz davon ist.

4. Chemiluminiszenz-Reagens gemäss Anspruch 3 welches das Natrium Salz ist.

5. Verwendung eines Chemiluminiszenz-Reagenzes gemäss eines der Ansprüche 2 bis 4 zur Bestimmung von aktivem Sauerstoff in lebenden Organismen.

6. Zwischenprodukt für die Herstellung von Chemiluminiszenz-Reagenzien gemäss Anspruch 2, welches eine Verbindung gemäss Anspruch 1 ist, wobei X Wasserstoff bedeutet.

7. Zwischenprodukt für die Herstellung von Chemiluminiszenz-Reagenzien gemäss Anspruch 2, welches eine Verbindung gemäss Anspruch 1 ist, wobei X eine Aminoschutzgruppe vom in Peptidsynthesen verwendeten Typ bedeutet.

8. Zwischenprodukt für die Herstellung von Chemiluminiszenz-Reagenzien gemäss Anspruch 2, welche eine Verbindung gemäss Anspruch 1 ist, wobei X eine Aminoschutzgruppe wie:
Benzyloxycarbonyl, p-Methoxybenzyloxycarbonyl, p-Chlorobenzyloxycarbonyl, p-Nitrobenzyloxycarbonyl, p-Phenylazobenzyloxycarbonyl, p-Methoxyphenyl-azobenzyloxycarbonyl, t-Butyloxycarbonyl, t-Amyloxycarbonyl, p-Biphenylisopropyloxycarbonyl, Diisopropylmethyloxycarbonyl und Formyl bedeutet.

9. Verwendung eines Zwischenproduktes gemäss eines der Ansprüche 6 bis 8 für die Herstellung eines Chemiluminiszenz-Reagenzes.

10. Verfahren für die Herstellung eines Zwischenproduktes gemäss Anspruch 7 oder Anspruch 8, das die Kondensation einer Verbindung der Formel (II): 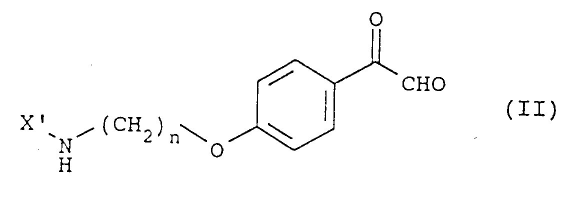 wobei X' eine Aminoschutzgruppe, wie in Anspruch 7 oder 8 definiert, und n eine Ziffer von 1 bis 4 bedeutet,
mit 2-Aminoacetamidin beinhaltet, um ein Pyrazin Derivat zu bilden und das resultierende Produkt mit Methylglyoxal umzusetzen, um ein entsprechendes 3,7-Dihydro[1,2-a]pyrazin-3-on gemäss Formel (I) zu erhalten, wobei X eine beschriebene Aminoschutzgruppe ist.

11. Verfahren zur Herstellung eines Zwischenproduktes gemäss Anspruch 6, das die Entfernung einer Aminosäureschutzgruppe einer Verbindung gemäss Anspruch 7 oder 8 beinhaltet.

12. Verfahren zur Herstellung eines Chemiluminiszenz-Reagenzes gemäss Anspruch 2, in dem eine Verbindung gemäss Anspruch 6 mit einem fluoreszierenden Reaktanden R-Y, R-CO-Y, R-SO₂-Y, R-NHCO-Y, oder R-NHCS-Y umgesetzt wird, wobei R eine wie in Anspruch 1 definierte fluoreszierende Gruppe und Y eine aminoreaktive funktionale Gruppe bedeutet.

## Patentansprüche (Patentansprüche für folgende(n) Vertragsstaat(en): ES, GR)

1. Verfahren zur Herstellung einer Verbindung der Formel (I): 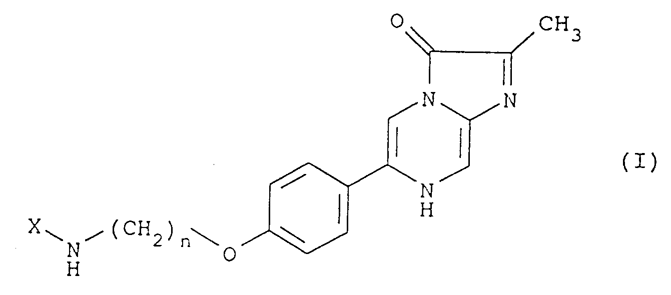 wobei X ein Wasserstoff, eine Aminoschutzgruppe vom in Peptidsynthesen verwendeten Typ, R-, R-CO-, R-SO₂-, R-NHCO-, oder R-NHCS-; R eine fluorenszierende Gruppe wie
5- oder 6-Carboxyfluoreszein-succinimidester,
5- oder 6-Carboxyfluoreszein-amidocapronsäure-N-hydroxysuccinimidester,
Fluoreszein-5-isocyanat,
Fluoreszein-5-isothiocyanat,
5-(4,6-Dichlorotriazin-2-yl)amino-fluoreszein dihydrochlorid,
4-Dimethylamino-azobenzyl-4'-sulfonylchlorid,
4-Dimethylamino-azobenzyl-4'-isothiocyanat,
4-Chloro-7-nitrobenz-2-oxa-1,3-diazol, und
4-Fluoro-7-nitrobenz-2-oxa-1,3-diazol,
und n ist eine Ziffer von 1 bis 4, oder Salze davon bedeutet;
welches
(i) die Kondensation einer Verbindung der folgenden Formel (II): 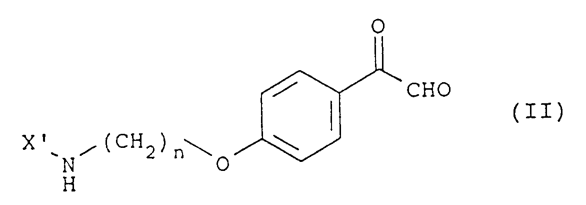 wobei X' eine Aminoschutzgruppe und n eine Ziffer von 1 bis 4 bedeutet, mit 2-Aminoacetamidin beinhaltet, um ein Pyrazinderivat zu bilden und das resultierende Produkt mit Methylglyoxal umzusetzen, um ein entsprechendes 3,7-Dihydro[1,2-a]pyrazin-3-on gemäss Formel (I) zu erhalten, wobei X eine beschriebene Aminoschutzgruppe bedeutet;
und
(ii) wenn erforderlich Umsetzung des Produkts mit einem fluoreszierenden Reaktanden R-Y, R-CO-Y, R-SO₂-Y, R-NHCO-Y, oder R-NHCS-Y beinhaltet, wobei R eine oben definierte fluoreszierende Gruppe und Y eine aminoreaktive funktionale Gruppe bedeutet.

2. Verfahren gemäss Anspruch 1 für die Herstellung eines Chemiluminiszenz-Reagenzes, das eine Verbindung gemäss Formel (I), worin X ein R-, R-CO-, R-SO₂-, R-NHCO-, oder R-NHCS-, und R eine beschriebene fluoreszierende Gruppe bedeutet, oder eines Salzes davon ist und worin Schritt (ii) ausgeführt wird.

3. Verfahren gemäss Anspruch 2 worin das beschriebene Chemiluminiszenz-Reagenz 3,7-Dihydro-6-(4-(2-(N'-(5-Fluoreszeinyl)-thioureido)ethoxy)-phenyl)-2-methylimidazo [1,2-a]pyrazin-3-on oder ein Salz davon ist.

4. Verfahren gemäss Anspruch 3 in welchem das Natriumsalz hergestellt wird.

5. Verfahren gemäss Anspruch 1 für die Herstellung eines Zwischenproduktes für die Produktion von Chemiluminiszenz-Reagenzien, in dem X in Formel (I) ein Wasserstoff bedeutet und Schritt (ii) nicht ausgeführt wird.

6. Verfahren gemäss Anspruch 1, in dem X in Formel (I) eine Aminoschutzgruppe vom in Peptidsynthesen verwendeten Typ bedeutet und Schritt (ii) nicht ausgeführt wird.

7. Verfahren gemäss Anspruch 6, in dem X eine Aminoschutzgruppe wie: Benzyloxycarbonyl, p-Methoxybenzyloxycarbonyl, p-Chlorobenzyloxycarbonyl, p-Nitrobenzyloxycarbonyl, p-Phenylazobenzyloxycarbonyl, p-Methoxyphenyl-azobenzyloxycarbonyl, t-Butyloxycarbonyl, t-Amyloxycarbonyl, p-Biphenylisopropyloxycarbonyl, Diisopropylmethyloxycarbonyl und Formyl. bedeutet.

8. Verfahren gemäss Anspruch 5, in dem die Aminoschutzgruppe einer Verbindung, hergestellt gemäss Anspruch 6 oder Anspruch 7, enfernt wird.

## Revendications (Revendications pour l'(les) Etat(s) contractant(s) suivant(s): AT, BE, CH, DE, DK, FR, GB, IT, LI, LU, NL, SE)

1. Composés représentés par la formule (I) ci-après : 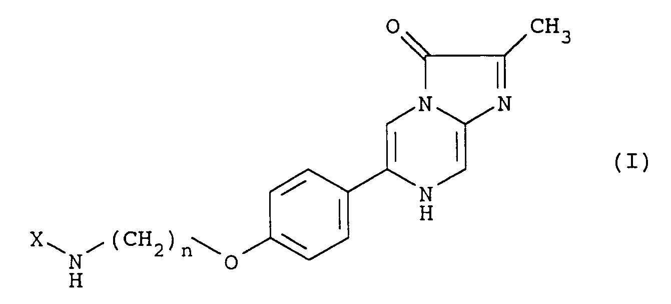 dans laquelle X est un hydrogène, un groupe amino-protecteur du type utilisé dans la synthèse des peptides, R-, R-CO-, R-SO₂-, R-NHCO-, ou R-NHCS-, R étant un groupe fluorescent choisi parmi
l'ester succinimidylique de la 5- ou 6-carboxyfluorescéine,
l'ester N-hydroxysuccinimidylique de l'acide 5- ou 6-carboxyfluorescéine-amidocaproïque,
le fluorescéine-5-isocyanate,
le fluorescéine-5-isothiocyanate,
le dichlorhydrate de 5-(4,6-dichlorotriazin-2-yl)aminofluorescéine,
le chlorure de 4-diméthylamino-azobenzène-4'-sulfonyle,
le 4-diméthylamino-azobenzène-4'-isothiocyanate,
le 4-chloro-7-nitrobenzo-2-oxa-1,3-diazole
et le 4-fluoro-7-nitrobenzo-2-oxa-1,3-diazole, et
n est un entier de 1 à 4, ou leurs sels.

2. Réactif chimioluminescent, qui est un composé selon la revendication 1, dans lequel X, dans la formule (I), est R-, R-CO-, R-SO₂-, R-NHCO-, ou R-NHCS-, R étant ledit groupe fluorescent, ou l'un de ses sels.

3. Réactif chimioluminescent selon la revendication 2, qui est la 3,7-dihydro-6-(4-(2-(N'-(5-fluorescéinyl)-thiouréido)éthoxy)phényl)-2-méthyl-imidazo[1,2-a]pyrazine-3-one, ou l'un de ses sels.

4. Réactif chimioluminescent selon la revendication 3, qui est le sel de sodium.

5. Utilisation d'un réactif chimioluminescent selon l'une quelconque des revendications 2 à 4, pour doser l'oxygène actif dans des organismes vivants.

6. Intermédiaire pour la production de réactifs chimioluminescents selon la revendication 2, qui est un composé selon la revendication 1 dans lequel X est un atome d'hydrogène.

7. Intermédiaire pour la production de réactifs chimioluminescents selon la revendication 2, qui est un composé selon la revendication 1 dans lequel X est un groupe amino-protecteur du type utilisé dans la synthèse peptidique.

8. Intermédiaire pour la production d'un réactif chimioluminescent selon la revendication 2, qui est un composé selon la revendication 1 dans lequel X est un groupe amino-protecteur du type choisi parmi les benzyloxycarbonyle,
p-méthoxybenzyloxycarbonyle,
p-chlorobenzyl-oxycarbonyle,
p-nitrobenzyloxycarbonyle,
p-phénylazobenzyloxycarbonyle,
p-méthoxyphényl-azobenzyloxycarbonyle,
t-butoxycarbonyle,
t-amyloxycarbonyle,
p-biphénylisopropyloxycarbonyle,
diisopropylméthyloxy-carbonyle et
formyle.

9. Utilisation d'un intermédiaire selon l'une quelconque des revendications 6 à 8 pour fabriquer un réactif chimioluminescent.

10. Procédé pour préparer un intermédiaire selon la revendication 7 ou la revendication 8, qui consiste à condenser un composé de formule (II) ci-après : 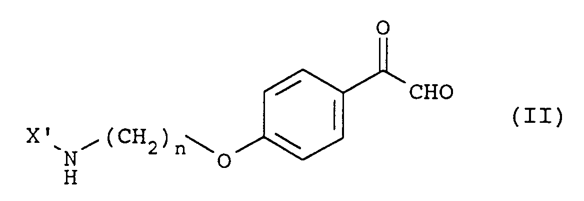 dans laquelle X' est un groupe amino-protecteur selon les revendications 7 ou 8 et n est un nombre entier ayant pour valeur 1 à 4,
avec la 2-aminoacétamidine pour former un dérivé de pyrazine, et à faire réagir le produit résultant ainsi obtenu avec du méthylglyoxal pour obtenir une 3,7-dihydro[1,2-a]pyrazine-3-one correspondante de formule (I) dans laquelle X est un groupe amino-protecteur du type ci-dessus.

11. Procédé pour préparer un intermédiaire selon la revendication 6, qui consiste à enlever un groupe amino-protecteur d'un composé selon la revendication 7 ou 8.

12. Procédé pour préparer un réactif de chimioluminescence selon la revendication 2, dans lequel on fait réagir un composé selon la revendication 6 avec un réactif fluorescent R-Y, R-CO-Y, R-SO₂-Y, R-NHCO-Y ou R-NHCS-Y, où R est un groupe fluorescent tel que défini dans la revendication 1, et Y est un groupe fonctionnel réactif vis-à-vis des amines.

## Revendications (Revendications pour l'(les) Etat(s) contractant(s) suivant(s): ES, GR)

1. Procédé pour la préparation d'un composé représenté par la formule (I) ci-après : 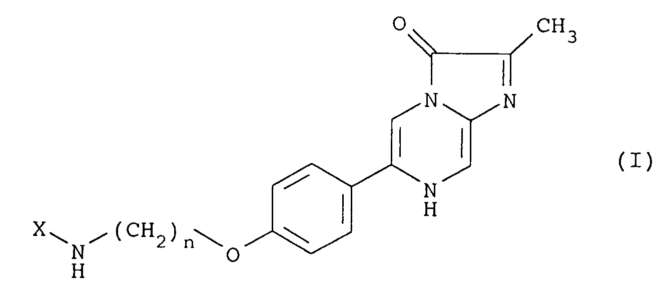 dans laquelle X est un hydrogène, un groupe amino-protecteur du type utilisé dans la synthèse peptidique, R-, R-CO-, R-SO₂-, R-NHCO-, ou R-NHCS-, R étant un groupe fluorescent choisi parmi
l'ester succinimidylique de la 5- ou 6-carboxyfluorescéine,
l'ester N-hydroxysuccinimidylique de l'acide 5- ou 6-carboxyfluorescéine-amidocaproïque,
le fluorescéine-5-isocyanate,
le fluorescéine-5-isothiocyanate,
le dichlorhydrate de 5-(4,6-dichlorotriazin-2-yl)aminofluorescéine,
le chlorure de 4-diméthylamino-azobenzène-4'-sulfonyle,
le 4-diméthylamino-azobenzène-4'-isothiocyanate,
le 4-chloro-7-nitrobenzo-2-oxa-1,3-diazole et
le 4-fluoro-7-nitrobenzo-2-oxa-1,3-diazole,
n est un entier de 1 à 4, ou leurs sels,
ledit procédé comprenant :
(i) la condensation d'un composé de formule (II) ci-après : 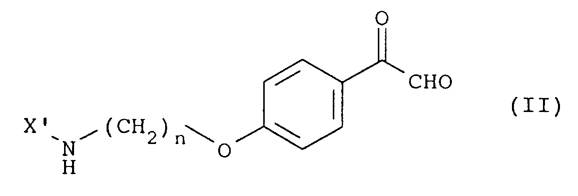 dans laquelle X' est un groupe amino-protecteur et n est un nombre entier ayant pour valeur 1 à 4,
avec la 2-aminoacétamidine pour former un dérivé de pyrazine, et la réaction du produit résultant ainsi obtenu avec le méthylglyoxal pour obtenir une 3,7-dihydro[1,2-a]pyrazine-3-one correspondante de formule (I) dans laquelle X est ledit groupe amino-protecteur, et
(ii) si nécessaire, la réaction du produit résultant avec un réactif fluorescent R-Y, R-CO-Y, R-SO₂-Y, R-NHCO-Y ou R-NHCS-Y, où R est un groupe fluorescent tel que défini ci-dessus, et Y est un groupe fonctionnel réactif vis-à-vis des amines.

2. Procédé suivant la revendication 1, pour préparer un réactif chimioluminescent, qui est un composé selon la revendication 1, dans lequel X, dans la formule (I), est R-, R-CO-, R-SO₂-, R-NHCO-, ou R-NHCS-, R étant ledit groupe fluorescent, ou l'un de ses sels, et dans lequel l'étape (ii) est effectuée.

3. Procédé selon la revendication 2, dans lequel le réactif chimioluminescent est la 3,7-dihydro-6-(4-(2-(N'-(5-fluorescéinyl)-thiouréido)éthoxy)phényl)-2-méthyl-imidazo[1,2-a]pyrazine-3-one, ou l'un de ses sels.

4. Procédé selon la revendication 3, dans lequel est préparé le sel de sodium.

5. Procédé suivant la revendication 1, pour la préparation d'un intermédiaire pour la production de réactifs chimioluminescents dans lequel X dans la formule (I) est l'atome hydrogène, et dans lequel l'étape (ii) n'est pas effectuée.

6. Procédé suivant la revendication 1, dans lequel X dans la formule (I) est un groupe amino-protecteur du type employé dans la synthése peptidique et dans lequel l'étape (ii) n'est pas effectuée.

7. Procédé suivant la revendication 6, dans lequel X est un groupe amino-protecteur choisi parmi les
benzyloxycarbonyle,
p-méthoxybenzyloxycarbonyle,
p-chlorobenzyloxycarbonyle,
p-nitrobenzyloxycarbonyle,
p-phényl-azobenzyloxycarbonyle,
p-méthoxyphényl-azobenzyloxycarbonyle,
t-butoxycarbonyle,
t-amyloxycarbonyle,
p-biphénylisopropyloxycarbonyle,
diisopropylméthyloxycarbonyle, et
formyle.

8. Procédé suivant la revdendication 5, dans lequel le groupe protecteur est éliminé d'un composé préparé selon la revendication 6 ou 7.
